(19) **Europäisches Patentamt / European Patent Office / Office européen des brevets**

(11) **EP 1 168 249 B1**

(12) # FASCICULE DE BREVET EUROPEEN

(45) Date de publication et mention
de la délivrance du brevet:
**26.10.2011 Bulletin 2011/43**

(51) Int Cl.:
***G06T 17/00*** *(2006.01)*

(21) Numéro de dépôt: **01401511.9**

(22) Date de dépôt: **11.06.2001**

(54) **Procédé et dispositif d'imagerie radiographique pour la reconstruction tridimensionnelle à faible dose d'irradiation**

Verfahren und Vorrichtung zur dreidimensionalen Rekonstruktion von Röntgenbilder mit niedriger Strahlungsdosis

Radiographic imaging method and device for three-dimensional reconstruction with low dose of irradiation

(84) Etats contractants désignés:
**CH DE GB IT LI SE**

(30) Priorité: **23.06.2000 FR 0008123**

(43) Date de publication de la demande:
**02.01.2002 Bulletin 2002/01**

(60) Demande divisionnaire:
**11151762.9 / 2 309 462**

(73) Titulaire: **EOS Imaging
75011 Paris (FR)**

(72) Inventeurs:
• **Dorion, Irène**
**75011 Paris (FR)**
• **Desaute, Pascal**
**75020 Paris (FR)**
• **Charpak, Georges**
**75005 Paris (FR)**
• **Skalli, Wafa**
**75013 Paris (FR)**
• **Veron, Stéphane**
**92130 Issy les Moulineaux (FR)**
• **Mitton, David**
**94270 Le Kremlin Bicetre (FR)**

• **De Guise, Jacques A.**
**Montreal, Quebec H2W 2L6 (CA)**
• **Landry, Champlain**
**Montreal, Quebec H2J 3K8 (CA)**

(74) Mandataire: **Burbaud, Eric
Cabinet Plasseraud
52 rue de la Victoire
75440 Paris Cedex 09 (FR)**

(56) Documents cités:
**EP-A2- 0 747 728    US-A- 4 053 779**

• **HUYNH T N ET AL: "DEVELOPMENT OF A VERTEBRAL ENDPLATE 3-D RECONSTRUCTION TECHNIQUE" IEEE TRANSACTIONS ON MEDICAL IMAGING,US, IEEE INC. NEW YORK, vol. 16, no. 5, 1 octobre 1997 (1997-10-01), pages 689-696, XP000722563 ISSN: 0278-0062**
• **D.MITTON ET AL: "3D reconstruction method from biplanar radiography using non-stereocorresponding points and elastic deformable meshes" MEDICAL & BIOLOGICAL ENGINEERING & COMPUTING 2000, vol. 38, mars 2000 (2000-03), pages 133-139, XP000913390**

**Description**

La présente invention est relative aux procédés et dispositifs d'imagerie radiographique pour la reconstruction tridimensionnelle à faible dose d'irradiation.

Plus particulièrement, l'invention concerne un procédé d'imagerie radiographique pour la reconstruction tridimensionnelle à faible dose d'irradiation, adapté pour calculer un modèle à trois dimensions d'au moins un objet prédéterminé à observer dans un champ d'observation, ce procédé comprenant les étapes suivantes :

(a) prendre au moins deux images radiographiques à deux dimensions du champ d'observation, respectivement selon deux directions de prise de vue non parallèles,

(b) repérer, sur chaque image radiographique, des repères de contrôle appartenant audit objet à observer,

(c) déterminer une position géométrique de chaque repère de contrôle dans un référentiel à trois dimensions,

(d) calculer la forme à trois dimensions d'un modèle représentant ledit objet à partir d'un modèle générique prédéterminé correspondant audit objet, ce modèle générique comportant des repères qui correspondent aux repères de contrôle identifiés sur les images radiographiques, le modèle calculé étant obtenu par déformation du modèle générique de façon que ledit modèle calculé suive une forme la plus proche possible d'une isométrie du modèle générique tout en maintenant en coïncidence les repères du modèle générique déformé avec les repères de contrôle reconstruits à l'étape (c).

Des procédés de reconstruction en trois dimensions du type susmentionné ont été divulgués notamment par Abdel-Aziz et al. ("Direct linear transformation from comparator coordinates into object space coordinates in close range photogrammetry", Prcc. ASP/UI Symp. Close Range Photogrammetry, Urbana, Illinois, USA, 1971; et Marzan ("Rational design for close range photogrammetry", PhD thesis, Department of Civil Engineering, University of Illinois, Urbana-Champaign, USA, 1976).

Dans ces procédés, tous les repères de contrôle sont des points de contrôle stéréo-correspondants et au cours de l'étape (b), on positionne ces repères dans l'espace au moyen d'un algorithme dit "transformation linéaire directe" (DLT), utilisé notamment par André et al. ("Optimized vertical stereo base radiographic setup for the clinical three-dimensional reconstruction of the human spine", J. Biomech., 27, pp 1023-1035, 1994). Par ailleurs, dans ces procédés connus, l'étape (d) est une étape de krigeage consistant en une interpolation/extrapolation du modèle générique de l'objet à observer, qui donne des positions estimées d'un grand nombre de repères du modèle à trois dimensions de l'objet à observer en fonction des coordonnées mesurées des repères de contrôle stéréo-correspondants et en fonction de la géométrie du modèle générique. Cette étape de krigeage a été décrite notamment par Trochu ("A contouring program based on dual kriging interpolation", Eng. Comput. 9, pp 160-177, 1993).

Ces procédés connus présentent l'avantage de permettre la réalisation d'un modèle tridimensionnel du ou des objets à observer, tout en permettant de réduire l'émission de rayonnements ionisants vers le champ d'observation par rapport à une information tridimensionnelle basée sur la reconstruction de coupes scanner telles que pratiquées dans les instrument actuels. Le modèle tridimensionnel peut ensuite être affiché sous différents angles de vue, par exemple sur un écran d'ordinateur.

Mais ces procédés souffrent d'un manque de précision et sont mal adaptés pour examiner convenablement un champ d'observation étendu tel que par exemple l'ensemble de la colonne vertébrale d'un patient.

Par ailleurs, MITTON et al. Medical & Biological Engineering & Computing 2000, Vol. 38, p 133-139) ont décrit un procédé de reconstruction tridimensionnelle qui utilise des repères non stéréo-correspondants en plus des repères stéréo-correspondants, pour reconstruire une image à trois dimensions d'une vertèbre isolée, à partir de deux images radiographiques de cette vertèbre prises successivement sous deux angles différents. Ce document a été utilisé pour délimiter la forme en deux portes des revendications 1 et 11.

Ce procédé n'est toutefois pas adapté pour une utilisation médicale, où il est nécessaire d'avoir une plus grande facilité de mise en oeuvre, une meilleure précision notamment sur des champs d'observation étendus, et une plus grande rapidité de mise en oeuvre.

La présente invention a notamment pour but de pallier ces inconvénients.

A cet effet, selon l'invention, un procédé du genre en question est **caractérisé en ce qu'**au cours de l'étape (a), les deux images radiographiques sont prises simultanément, par balayage, en déplaçant en synchronisme, dans une même direction de translation non parallèle aux directions de prises de vues, au moins une source radioactive émettant deux faisceaux de rayons ionisants respectivement dans les deux directions de prise de vue.

Le champ d'observation mentionné ci-dessus peut comprendre notamment le rachis, le bassin, ou encore le genou d'un patient, ou plus généralement être constitué par tout ou partie du squelette du patient. Dans ces différents cas, les objets à observer peuvent être constitués notamment par les os du patient compris dans le champ d'observation ainsi que la durée des opérations de prise de vue.

Grâce aux dispositions susmentionnées, on obtient une bonne précision de la reconstruction à trois dimensions, y compris pour des champs d'observation étendus, et ce en limitant la dose de rayonnements émise vers le champ d'observation.

Cette précision est obtenue grâce à la simultanéité des deux prises de vues, et grâce à la prise de vue par balayage qui améliore la précision dans la direction du balayage notamment pour les champs d'observation étendus.

Dans des modes de réalisation préférés du procédé selon l'invention, on peut éventuellement avoir recours en cutre à l'une et/ou à l'autre des dispositions suivantes :

- au cours de l'étape (b), certains des repères de contrôle identifiés sont des repères de contrôle non stéréo-correspondants visibles et identifiés sur une seule image, et au cours de l'étape (c), la position géométrique de chaque repère de contrôle non stéréo-correspondant dans le référentiel à trois dimensions est estimée à partir du modèle générique, en déplaçant les repères correspondant aux repères de contrôle non stéréo-correspondants du modèle générique chacun sur une droite joignant :

    · d'une part, la source radioactive à l'origine de l'image radiographique dans laquelle une projection de ce repère de contrôle non stéréo-correspondant est visible et identifiable (la source radioactive étant positionnée sur l'emplacement de sa trajectoire qui correspond à la prise d'image où est visible ledit repère de contrôle),
    · et d'autre part, ladite projection de ce repère sur l'image radiographique,

    les repères de contrôle non stéréo-correspondants étant ainsi déplacés jusqu'à des positions respectives qui minimisent la déformation globale du modèle générique de l'objet à observer ;

- au cours de l'étape (c), on minimise la valeur de la somme quadratique :

$$S = \lambda . \sum_{i=1}^{m} k_i . (x_i - x_{i0})^2 ,$$

    où λ est un coefficient constant, m est un nombre entier de ressorts fictifs reliant chaque repère du modèle générique à d'autres reperes de ce modèle, k est une valeur de raideur prédéterminée du ressort fictif d'indice i, $x_i$ est la longueur du ressort fictif d'indice i dans le modèle générique initial, et $x_i$ et la longueur du ressort fictif d'indice i dans le modèle générique déformé (d'autres équations pour exprimer des déformations de lignes, surfaces ou volumes peuvent être envisagées);

- au cours de l'étape (b), certains des repères de contrôle identifiés sont des repères de contrôle stéréo-correspondants visibles et identifiés sur les deux images, et au cours de l'étape (c), la position géométrique des repères de contrôle stéréo-correspondants est directement calculée à partir de mesures de position des projections de ces repères sur les deux images ;

- au cours de l'étape (b), on repère sur chaque image radiographique des lignes de contour correspondant à des limites de l'objet observé et/ou à des lignes de plus grande densité optique à l'intérieur desdites limites, ces lignes de contour comprenant des projections des repères de contrôle sur les images radiographiques ;

- au cours de l'étape (c), on détermine des repères du modèle générique correspondant aux repères de contrôle, lesdits repères du modèle générique comprenant des portions dudit modèle générique qui se présentent tangentiellement par rapport aux rayons issus des sources radioactives et ayant généré les images radiographiques ;

- l'étape (c) comporte les sous-étapes suivantes :

    (c1) créer un modèle générique recalé en adaptant la taille du modèle générique et la position de ce modèle générique dans le référentiel, pour que les projections respectives du modèle générique recalé à partir des deux sources radioactives correspondent sensiblement aux deux images radiographiques,
    (c2) sélectionner des repères du modèle générique dont les projections sur au moins une des images radiographiques à partir de la source radioactive correspondante, sont les plus proches des lignes de contour repérées au cours de l'étape (b),
    (c3) définir une surface enveloppe formée par des rayons issus de chaque source radioactive et ayant contribué à générer lesdites lignes de contour des images radiographiques,
    (c4) déterminer certains repères du modèle générique recalé correspondant à des surfaces dudit modèle générique recalé, qui sont tangentes auxdites surfaces enveloppes, les repères du modèle générique recalé ainsi déterminés correspondant aux repères de contrôle,
    (c5) et déterminer la position géométrique de chaque repère de contrôle par projection du repère correspondant du modèle générique recalé sur la surface enveloppe correspondante ;

- les deux directions de prise de vue sont perpendiculaires l'une à l'autre ;
- on fait émettre par chacune des sources radioactives un faisceau de rayonnements ionisants dans un plan perpendiculaire à la direction de translation ;
- les deux faisceaux de rayons ionisants sont émis respectivement par deux sources radioactives.

Par ailleurs, l'invention a également pour objet un dispositif d'imagerie radiographie pour la mise en oeuvre d'un procédé tel que défini ci-dessus, ce dispositif comportant :

- des moyens d'émission de rayons ionisants comprenant au moins une source radioactive, ces moyens d'émission étant adaptés pour émettre respectivement deux faisceaux de rayonnements ionisants vers un champ d'observation contenant au moins un objet à observer, dans deux directions de prise de vue non parallèles, lesdits moyens d'émission étant déplaçables simultanément selon une direction de translation non parallèle aux directions de prise de vue,
- au moins deux dispositifs de détection disposes respectivement face aux deux faisceaux de rayons ionisants, au-delà du champ d'observation, pour mesurer les rayonnements ionisants ayant traversé ledit champ d'observation, ces deux dispositifs de détection étant déplaçables en synchronisme avec les moyens d'émission dans ladite direction de translation,
- des moyens pour prendre simultanément au moins deux images radiographiques à deux dimensions du champ d'observation, par balayage simultané du champ d'observation avec les sources radioactives et les détecteurs dans la direction de translation,
- des moyens d'identification pour identifier, sur chaque image radiographique, des repères de contrôle prédéterminés appartenant audit objet à observer,
- des premiers moyens de reconstruction pour déterminer une position géométrique de chaque repère de contrôle dans un référentiel à trois dimensions, à partir d'un modèle générique prédéterminé correspondant audit objet, ce modèle générique comportant des repères qui correspondent aux repères de contrôle identifiés sur les images radiographiques,
- et des seconds moyens de reconstruction pour calculer la forme à trois dimensions d'un modèle représentant ledit objet à partir du modèle générique, lesdits seconds moyens de reconstruction étant adaptés pour déterminer le modèle calculé par déformation du modèle générique de façon que ledit modèle calculé suive une forme la plus proche possible d'une isométrie du modèle générique tout en maintenant en coïncidence les repères du modèle générique déformé avec les repères de contrôle reconstruits par les premiers moyens de reconstruction.

Dans des modes de réalisation préférés du dispositif selon l'invention, on peut éventuellement avoir recours en outre à l'une et/cu à l'autre des dispositions suivantes :

- les moyens d'identification sont adaptés pour identifier au moins certains repères de contrôle visibles et identifiables sur une seule image radiographique

dits repères de contrôle non stéréo-correspondants et les premiers moyens de reconstruction sont adaptés pour estimer la position géométrique des repères de contrôle non stéréo-correspondants en déplaçant les repères du modèle générique correspondant aux repères de contrôle non stéréo-correspondants, chacun sur une droite joignant :

. d'une part, la source radioactive à l'origine de l'image radiographique où une projection de ce repère de contrôle non stéréo-correspondant est visible et identifiable,
. et d'autre part, ladite projection de ce repère sur l'image radiographique,

les premiers moyens de reconstruction étant adaptés pour déplacer ainsi les repères de contrôle non stéréo-correspondants jusqu'à des positions respectives qui minimisent la déformation globale du modèle générique de l'objet à observer ;
- les moyens d'identification sont adaptés pour identifier sur les deux images des repères de contrôle qui sont visibles et identifiables sur lesdites deux images, dits repères de contrôle stéréo-correspondants, et les premiers moyens de reconstruction sont adaptés pour déterminer la position géométrique des repères de contrôle stéréo-correspondants par calcul à partir de mesures de position des projections de ces repères sur les deux images ;
- chaque détecteur comprend une ligne de cellules de détection perpendiculaire à la direction de translation, les faisceaux de rayonnements ionisants étant perpendiculaires à ladite la direction de translation ;
- les moyens d'émission et les détecteurs sont déplaçables sur une distance de balayage d'au moins 7C cm ;
- les moyens d'émission comprennent deux sources radioactives à l'origine respectivement des deux faisceaux de rayons ionisants.

D'autres caractéristiques et avantages de l'invention apparaitront au cours de la description suivante d'une de ses formes de réalisation, donnée à titre d'exemple non limitatif, en regard des dessins joints.
Sur les dessins :

- la figure 1 est une vue schématique d'un appareil de radiographie selon une forme de réalisation de l'invention, permettant d'effectuer simultanément une prise de vue de face et une prise de vue de profil du patient,
- la figure 2 est une vue schématique en perspective d'une vertèbre d'un patient examiné au moyen de l'appareil de la figure 1,
- les figures 3 et 4 sont respectivement des vues de profil et de face de la vertèbre de la figure 2, schématisant une partie des vues de profil et de face obtenues au moyen de l'appareil de la figure 1,

- et la figure 5 est une vue en perspective représentant un modèle à trois dimensions de la colonne vertébrale et du bassin du patient examiné au moyen de l'appareil de la figure 1, ce modèle étant calculé à partir des vues de profil et de face prises au moyen de l'appareil de la figure 1.

Sur les différentes figures, les mêmes références désignent des éléments identiques ou similaires.

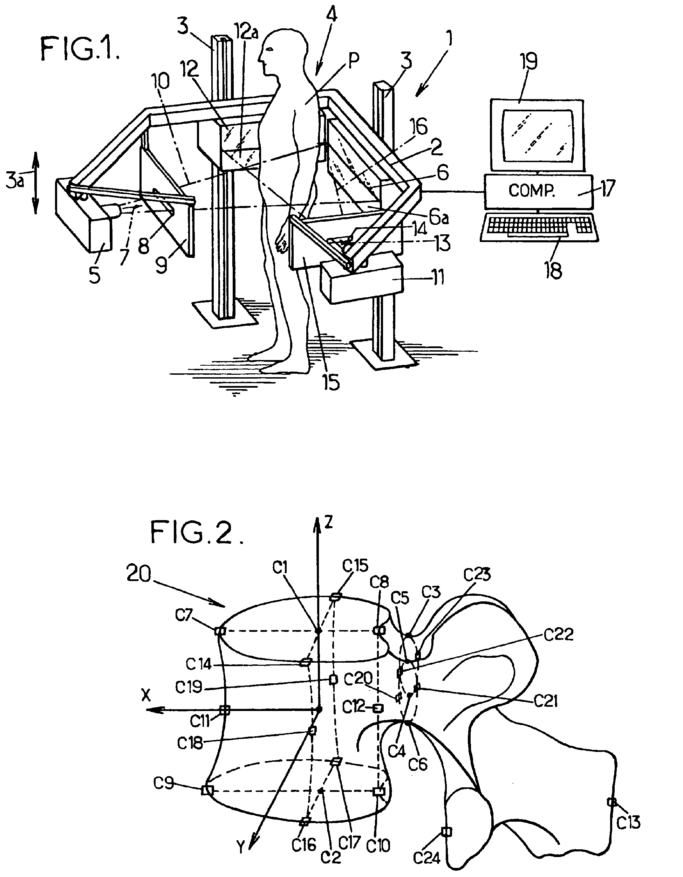

La figure 1 représente un dispositif radiographique 1 pour la reconstruction tridimensionnelle, comportant un bâti mobile 2 déplaçable verticalement de façon motorisée sur des guides verticaux 3, dans une direction de translation 3a.

Ce bâti entoure un champ d'observation 4 dans lequel peut prendre place un patient P debout. On peut ainsi observer la position des os du squelette de ce patient en station debout, ce qui est essentiel notamment pour les patients atteints de scoliose.

Le bâti mobile 2 porte une première source radioactive 5 et un premier détecteur 6 qui est disposé face à la source 5 au-delà du champ 4, et qui comporte une ligne horizontale 6a de cellules de détection. Le détecteur 6 peut par exemple être un détecteur gazeux sensible aux basses doses de rayonnements, par exemple tel que décrit dans le document FR-A-2 749 402 ou FR-A-2 754 068. Bien entendu, d'autres types de détecteurs, gazeux ou non, pourraient éventuellement être utilisés dans le cadre de la présente invention.

La source radioactive 5 est adaptée pour émettre des rayons ionisants, notamment des rayons X, dans une direction de prise de vue 7 antéro-postérieure par rapport au patient P, en traversant une fente horizontale 8 ménagée dans un réticule 9 tel qu'une plaque métallique, pour générer un faisceau horizontal 10 de rayonnements ionisants dans le champ d'observation 4.

Par ailleurs, le bâti mobile 2 porte également une deuxième source radioactive 11 similaire à la source 5 et un deuxième détecteur 12 similaire au détecteur 6, qui est disposé face à la source 11 au-delà du champ 4, et qui comporte une ligne horizontale 12a de cellules de détection.

La source radioactive 11 est adaptée pour émettre des rayons ionisants, dans une direction de prise de vue latérale 13 par rapport au patient P, en traversant une fente horizontale 14 ménagée dans un réticule 15 tel qu'une plaque métallique, pour générer un faisceau horizontal 16 de rayonnements ionisants dans le champ d'observation 4.

On notera que les sources radioactives et les détecteurs pourraient le cas échéant être en nombre supérieur à 2, et que les directions de prises de vue de ces différentes sources radioactives pourraient le cas échéant ne pas être perpendiculaires entre elles ni horizontales.

Les deux détecteurs 6, 12 sont reliés à un micro-ordinateur 17 ou autre système électronique de commande, équipé :

- d'une interface d'entrée comprenant au moins un clavier et généralement une souris (non représentée),
- et d'une interface de sortie comprenant au moins un écran 19 et généralement une imprimante (non représentée).

Le micro-ordinateur 17 peut également être relié aux moyens d'entraînement motorisés (non représentés) contenus dans les guides 3 et aux sources 5, 11, de façon à commander le déplacement vertical du bâti 2 et l'émission des rayonnements ionisants.

Le dispositif qui vient d'être décrit fonctionne comme suit.

Au moyen du micro-ordinateur 17, on fait d'abord prendre deux images radiographiques du patient P, en faisant balayer le champ d'observation 4 par les faisceaux 1C, 16 de rayonnements ionisants sur la hauteur correspondant à la zone du patient à observer, par exemple le rachis et le bassin, voire l'ensemble du squelette (à cet effet, le bâti est de préférence déplaçable sur une hauteur d'au moins 70 cm, voire supérieure à 1 m).

Au cours de ce mouvement, on enregistre dans la mémoire du micro-ordinateur 17 deux images radiographiques numériques, par exemple respectivement antéro-postérieure et latérale de la partie examinée du patient, lesquelles images peuvent être visualisées sur l'écran 19 du micro-ordinateur.

Chacune de ces images comprend généralement plusieurs objets prédéterminés à examiner, par exemple des vertèbres 20 telles que celle représentée schématiquement sur la figure 2.

Pour chacun de ces objets à examiner, le micro-ordinateur 17 a en mémoire un modèle générique à trois dimensions qui correspond à une forme moyenne de l'objet en question, lequel modèle générique est élaboré à l'avance par des méthodes statistiques en analysant un grand nombre d'objets similaires.

Lors de l'affichage des images radiographiques sur l'écran 19 du micro-ordinateur 17, le praticien peut par exemple indiquer au micro-ordinateur, notamment au moyen du clavier 18 ou de la souris, le type de chaque objet à examiner visible sur lesdites images, de façon que le micro-ordinateur 17 détermine le modèle générique correspondant à cet objet.

Par ailleurs, les modèles génériques utilisés pourraient également être constitués par des modèles préalablement réalisés par imagerie médicale sur le patient : dans ce cas, le procédé selon l'invention peut permettre par exemple de suivre l'évolution ultérieure du patient par des moyens plus simples, moins coûteux et émettant moins de radiations que les moyens d'imagerie tridimensionnelle classiques.

Le modèle générique de chaque objet, par exemple de chaque vertèbre 20 d'un squelette humain, comprend :

- les coordonnées d'une pluralité de repères de contrôle, notamment des points correspondant à des repères singuliers de cette vertèbre,

- et les coordonnées d'un grand nombre d'autres repères de l'objet en question, par exemple au nombre d'environ 200 ou plus.

Ces coordonnées peuvent être exprimées par exemple dans un référentiel local X, Y, Z. Dans l'exemple considéré, l'axe Z correspond à la direction "axiale" de la colonne vertébrale, l'axe X est déterminé de façon à définir avec l'axe Z le plan antéro-postérieur de la vertèbre 20, l'axe Y étant perpendiculaire aux axes X, Z susmentionnés. De plus, l'origine O du référentiel X, Y, Z est disposée au milieu des deux faces d'extrémité axiales de la partie principale "tubulaire" de la vertèbre, l'origine O étant par ailleurs positionnée pour que l'axe Z traverse la face axiale supérieure de la partie principale de la vertèbre en un repère C1 tel que la distance de ce repère C1 à l'extrémité avant C7 de ladite face axiale soit égale à environ 2/3 de la distance totale entre les extrémités avant C7 et arrière C8 de la section antéro-postérieure de ladite face axiale supérieure.

Les différents repères de contrôle C1-C25 susmentionnés se répartissent en deux catégories :

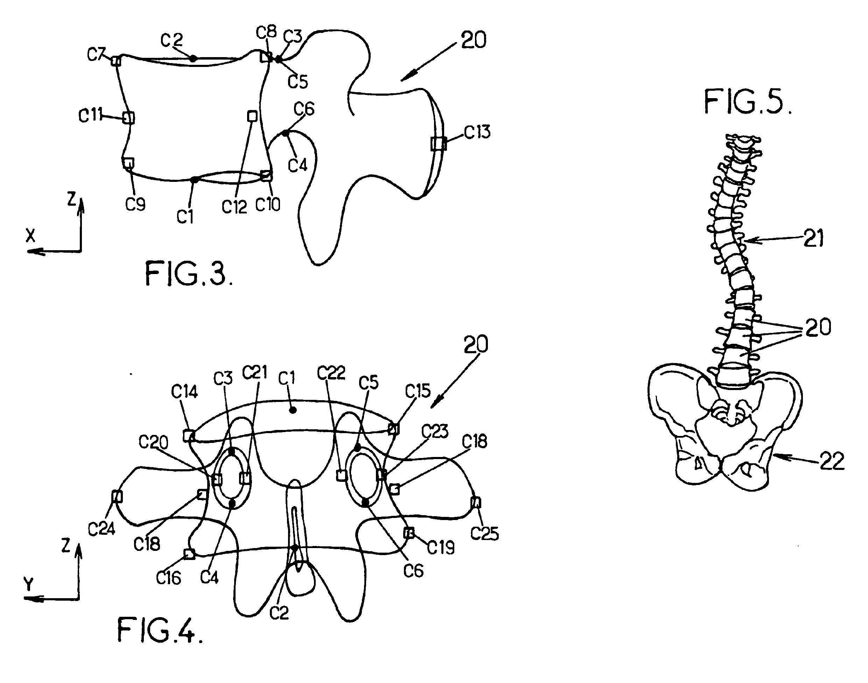

- des repères de contrôle "stéréo-correspondants" C1-C6, visibles et identifiables à la fois sur l'image radiographique latérale et sur l'image antéro-postérieure, ces repères étant au nombre de 6 dans l'exemple considéré (voir figures 3 et 4),
- et des repères de contrôle "non stéréo-correspondants" C7-C25, visibles et identifiables sur une seule image, ces repères étant au nombre de 19 dans l'exemple considéré.

Le praticien identifie ces différents repères de contrôle pour chaque objet à examiner (par exemple les vertèbres et le bassin) sur chaque image radiographique, par exemple en "marquant" ces repères à l'écran 19 par sélection au moyen de la souris et/ou du clavier. De plus, les deux images sont calibrées, de façon à pouvoir mesurer précisément la position de chaque repère de ces images dans un référentiel commun.

Ensuite, on détermine une position géométrique de chaque repère de contrôle de chaque objet, dans un référentiel à trois dimensions, par exemple le référentiel X, Y, Z susmentionné ou un référentiel commun à l'ensemble des objets à examiner.

La position des repères de contrôle stéréo-correspondants C1-C6 est directement calculée à partir de la mesure de la position de ces points sur les deux images.

De plus, la position géométrique de chaque repère de contrôle non stéréo-correspondant C7-C25 dans le référentiel à trois dimensions est estimée à partir du modèle générique, en déplaçant chaque repère de contrôle stéréo-correspondant C1-C6 du modèle générique jusqu'à sa position mesurée, et en déplaçant les repères de contrôle non stéréo-correspondants C7-C25 du modèle générique, chacun sur une droite joignant :

. d'une part, la source radioactive 5, 6 à l'origine de l'image radiographique où une projection de ce repère de contrôle non stéréo-correspondant est visible et identifiable,
. et d'autre part, ladite projection de ce repère sur l'image radiographique,

les repères de contrôle non stéréo-correspondants étant ainsi déplacés jusqu'à des positions respectives qui minimisent la déformation globale du modèle générique de l'objet à observer.

En particulier, on peut minimiser ladite déformation en minimisant (par exemple au moyen d'une méthode de gradient) la valeur de la somme quadratique :

$$S = \frac{1}{2} \cdot \sum_{i=1}^{m} k_i \cdot (x_i - x_{i0})^2 \, ,$$

ou plus généralement

$$S = \lambda \cdot \sum_{i=1}^{m} k_i \cdot (x_i - x_{i0})^2 \, ,$$

où $\lambda$ est un coefficient constant prédéterminé, m est un nombre entier non nul représentant un nombre de ressorts fictifs qui relient chaque repère de contrôle du modèle générique à d'autres repères de contrôle, k. est un coefficient de raideur prédéterminé du ressort fictif d'indice i, $x_{i,0}$ est la longueur du ressort fictif d'indice i dans le modèle générique non déformé, et $x_i$ est la longueur au ressort fictif d'indice i dans le modèle générique déformé.

Enfin, on calcule la forme à trois dimensions d'un modèle représentant la vertèbre 20 du patient, le modèle calculé étant obtenu par déformation du modèle générique de façon à maintenir la coïncidence des points de contrôle du modèle générique déformé avec la position spatiale précédemment déterminée des points de contrôle et de façon que ledit modèle calculé suive une forme la plus proche possible d'une isométrie du modèle générique, en travaillant cette fois sur l'ensemble des points de modèle générique.

En particulier, l'obtention du modèle à trois dimensions de chaque objet à examiner peut être obtenu par le procédé connu de krigeage ("krigirg").

Après le calcul du modèle à trois dimensions des différents objets à examiner, le micro-ordinateur 17 peut assembler la totalité des modèles à trois dimensions des différents objets à examiner, en fonction de la position de ces différents modèles dans un référentiel absolu commun à tous ces objets, de façon à obtenir un modèle à trois dimensions comprenant par exemple l'ensemble du rachis 21 du patient et le bassin 22 de ce patient, comme représenté sur la figure 5.

Une fois élaboré, ce modèle à trois dimensions peut être présenté sur l'écran 19 du micro-ordinateur, ou imprimé, sous l'angle de vision voulu. Ce modèle d'ensemble peut également être mis en mouvement à l'écran en fonction des commandes du praticien.

Le praticien dispose ainsi d'un outil efficace d'examen pouvant servir à l'imagerie de toute partie notamment osseuse ou cartilagineuse du corps humain ou animal, et utile notamment pour le diagnostic des scolioses ou pour le suivi pré ou post-opératoire lors d'interventions chirurgicales.

Bien entendu, on peut également calculer certains indices cliniques prédéterminés liés soit à la géométrie de l'ensemble examiné, soit le cas échéant à la composition ou la densité des objets à examiner, estimées à partir des images radiographiques (cas de l'ostéoporose par exemple).

On notera que le dispositif radiographique 1 pourrait le cas échéant être adapté pour l'examen d'un patient couché, ce qui peut s'avérer indispensable dans le domaine de la traumatologie. Dans ce cas, le patient P serait couché sur une table support, les faisceaux de rayons ionisants 10, 16 seraient chacun dans un plan vertical, et les sources 5, 11 se déplaceraient horizontalement avec les détecteurs 6, 12.

Par ailleurs, il va de soi que dans tous les cas, le dispositif radiographique 1 peut être utilisé également en radiographie à deux dimensions, en plus de son utilisation en imagerie tridimensionnelle.

On notera que le dispositif selon l'invention pourrait le cas échéant être utilisé dans des applications de radiologie non médicale.

Par ailleurs, au lieu d'utiliser des repères de contrôle C1-C25 définis à l'avance sur chaque modèle générique, il serait possible de déterminer et de positionner dans l'espace les repères de contrôle à partir de lignes de contour de l'objet à observer visibles sur l'une ou l'autre des deux images radiographiques.

A cet effet, on pourrait en particulier procéder comme suit :

- on repère sur chaque image radiographique des lignes de contour correspondant à des limites de l'objet observé et/ou à des lignes de plus grande densité optique à l'intérieur desdites limites,
- on crée un modèle générique recalé en adaptant la taille du modèle générique et la position de ce modèle générique dans le référentiel X, Y, Z pour que les projections respectives du modèle générique recalé à partir des deux sources radioactives 5, 11 correspondent sensiblement aux deux images radiographiques,

- on sélectionne des repères du modèle générique dont les projections sur au moins une des images radiographiques à partir de la source radioactive correspondante, sont les plus proches des lignes de contour repérées au cours de l'étape (b),

- on définit une surface enveloppe formée par des rayons issus de chaque source radioactives et ayant contribué à générer lesdites lignes de contour des images radiographiques,
- on détermine certains repères du modèle générique recalé correspondant à des surfaces dudit modèle générique recalé, qui sont tangentes auxdites surfaces enveloppes, les repères du modèle générique recalé ainsi déterminés correspondant aux repères de contrôle,
- on détermine la position géométrique de chaque repère de contrôle par projection dudit repère de contrôle sur la surface enveloppe correspondante,
- puis on procède par exemple comme décrit précédemment pour reconstituer un modèle à trois dimensions de l'ensemble de l'objet à observer, notamment par krigeage.

Cn notera enfin qu'il serait possible de générer deux faisceaux ionisants non parallèles au moyen de deux réticules (par exemple deux fentes distinctes mémorisées dans une même plaque métallique) à partir d'une source radioactive unique pour mettre en oeuvre l'invention, en utilisant comme précédemment deux détecteurs disposés face aux deux faisceaux et déplaçables en synchronisme avec la source et les réticules.

**Revendications**

1. Procédé d'imagerie radiographique pour la reconstruction tridimensionnelle à faible dose d'irradiation, adapté pour calculer un modèle à trois dimensions d'au moins un objet prédéterminé (20) à observer dans un champ d'observation (4) , ce procédé comprenant les étapes suivantes :

   (a) prendre au moins deux images radiographiques à deux dimensions du champ d'observation, respectivement selon deux directions de prise de vue (7, 13) non parallèles,
   (b) repérer, sur chaque image radiographique, des repères de contrôle (C1-C25) appartenant audit objet à observer,
   (c) déterminer une position géométrique de chaque repère de contrôle, dans un référentiel à trois dimensions,
   (d) calculer la forme à trois dimensions d'un modèle représentant ledit objet à partir d'un modèle générique prédéterminé correspondant audit objet, ce modèle générique comportant des repères qui correspondent aux repères de contrôle identifiés sur les images radiographiques, le modèle calculé étant obtenu par déformation du modèle générique de façon que ledit modèle calculé suive une forme la plus proche possible d'une iscmétrie du modèle générique tout en maintenant en coïncidence les repères du modèle générique déformé avec les repères de contrôle reconstruits à l'étape (c),

**caractérisé en ce qu'au** au cours de l'étape (a), les deux images radiographiques sont prises simultanément, par balayage, en déplaçant en synchronisme, dans une même direction de translation (3a) non parallèle aux directions de prises de vues, au moins une source radioactive (5, 11) émettant deux faisceaux de rayons ionisants (10, 16) respectivement dans les deux directions de prise de vue (7, 13).

2. Procédé selon la revendication 1, dans lequel :

- au cours de l'étape (b), certains des repères de contrôle identifiés, dits repères de contrôle non stéréo-correspondants ne sont visibles et identifiés que sur une seule image,
- et au cours de l'étape (c), la position géométrique de chaque repère de contrôle non stéréo-correspondant (C7-C25) dans le référentiel à trois dimensions est estimée à partir du modèle générique, en déplaçant les repères de contrôle non stéréo-correspondants du modèle générique, chacun sur une droite joignant :

. d'une part, la source radioactive (5, 11) à l'origine de l'image radiographique dans laquelle une projection de ce repère de contrôle non stéréo-correspondant est visible et identifiable,
. et d'autre part, ladite projection de ce repère sur l'image radiographique,

les repères de contrôle non stéréo-correspondants (C7-C25) étant ainsi déplacés jusqu'à des positions respectives qui minimisent la déformation globale du modèle générique de l'objet à observer.

3. Procédé selon la revendication 2, dans lequel au cours de l'étape (c), on minimise la valeur de la somme quadratique :

$$S = \lambda . \sum_{i=1}^{m} k_i . (x_i - x_{i0})^2 ,$$

où $\lambda$ est un coefficient constant, m est un nombre entier de ressorts fictifs reliant chaque repère (C1-C25) du modèle générique à d'autres repères de ce modèle, $k_i$ est une valeur de raideur prédéterminée du ressort fictif d'indice i, $x_{i0}$ est la longueur du ressort fictif d'indice i dans le modèle générique initial, et x :

est la longueur de ressort fictif d'indice i dans le modèle générique déformé.

4. Procédé selon l'une quelconque des revendications précédentes, dans lequel :

- au cours de l'étape (b), au moins certains des repères de contrôle identifiables sont des repères de contrôle stéréo-correspondants (C1-C6) visibles et identifiés sur les deux images,
- et au cours de l'étape (c), la position géométrique des repères de contrôle stéréo-correspondants (C1-C6) est directement calculée à partir de mesures de position des projections de ces repères sur les deux images.

5. Procédé selon la revendication 1, dans lequel au cours de l'étape (b), on repère sur chaque image radiographique des lignes de contour correspondant à des limites de l'objet observé et/ou à des lignes de plus grande densité optique à l'intérieur desdites limites, ces lignes de contour comprenant des projections des repères de contrôle sur les images radiographiques.

6. Procédé selon la revendication 5, dans lequel au cours de l'étape (c), on détermine des repères du modèle générique correspondant aux repères de contrôle, lesdits repères du modèle générique comprenant des portions dudit modèle générique qui se présentent tangentiellement par rapport aux rayons issus des sources radioactives et ayant généré les images radiographiques.

7. Procédé selon la revendication 6, dans lequel l'étape (c) comporte les sous-étapes suivantes :

(c1) créer un modèle générique recalé en adaptant la taille du modèle générique et la position de ce modèle générique dans le référentiel, pour que les projections respectives du modèle générique recalé à partir des deux sources radioactives correspondent sensiblement aux deux images radiographiques,
(c2) sélectionner des repères du modèle générique dont les projections sur au moins une des images radiographiques à partir de la source radioactive correspondante, sont les plus proches des lignes de contour repérées au cours de l'étape (b),
(c3) définir une surface enveloppe formée par des rayons issus de chaque source radioactive (5, 11) et ayant contribué à générer lesdites lignes de contour des images radiographiques,
(c4) déterminer certains repères du modèle générique recalé correspondant à des surfaces dudit modèle générique recalé, qui sont tangentes auxdites surfaces enveloppes, les repères du modèle générique recalé ainsi déterminés correspondant aux repères de contrôle,

(c5) et déterminer la position géométrique de chaque repère de contrôle par projection du repère correspondant du modèle générique recalé, sur la surface enveloppe correspondante.

8. Procédé selon l'une quelconque des revendications précédentes, dans lequel les deux directions de prise de vue (7, 13) sont perpendiculaires l'une à l'autre.

9. Procédé selon la revendication 8, dans lequel on fait émettre par chacune des sources radioactives (5, 11) un faisceau de rayonnements ionisants (10, 16) dans un plan perpendiculaire à la direction de translation (3a).

10. Procédé selon l'une quelconque des revendications précédentes, dans lequel les deux faisceaux de rayons ionisants (10, 16) sont émis respectivement par deux sources radioactives (5, 11).

11. Dispositif d'imagerie radiographie pour la mise en oeuvre d'un procédé selon l'une quelconque des revendications précédentes, ce dispositif comportant :

- des moyens (17) pour prendre au moins deux images radiographiques à deux dimensions du champ d'observation,
- des moyens d'identification (17) pour identifier, sur chaque image radiographique, des repères de contrôle prédéterminés (C1-C25) appartenant audit objet à observer,
- des premiers moyens de reconstruction (17) pour déterminer une position géométrique de chaque repère de contrôle dans un référentiel à trois dimensions (X, Y, Z), à partir d'un modèle générique prédéterminé correspondant audit objet, ce modèle générique comportant des repères qui correspondent aux repères de contrôle identifiés sur les images radiographiques,
- et des seconds moyens de reconstruction (17) pour calculer la forme à trois dimensions d'un modèle représentant ledit objet à partir du modèle générique, lesdits seconds moyens de reconstruction étant adaptés pour déterminer le modèle calculé par déformation du modèle générique de façon que ledit modèle calculé suive une forme la plus proche possible d'une isométrie du modèle générique tout en maintenant en coïncidence les repères (C1-C25) du modèle générique déformé avec les repères de contrôle reconstruits par les premiers moyens de reconstruction

**caractérisé en ce que** le dispositif comporte:

- des moyens d'émission de rayons ionisants comprenant au moins une source radioactive (5, 11), ces moyens d'émission étant adaptés pour émettre respectivement deux faisceaux de rayonnements ionisants (10, 16) vers un champ d'observation (4) contenant au moins un objet (20) à observer, dans deux directions de prise de vue (7, 13) non parallèles, lesdits moyens d'émission étant déplacables selon une direction de translation (3a) non parallèle aux directions de prise de vue de façon que les deux faisceaux (10, 16) balaient simultanément le champ d'observation,

- au moins deux dispositifs de détection (6, 12) disposés respectivement face aux deux faisceaux de rayons ionisants (10, 16), au-delà du champ d'observation (4), pour mesurer les rayonnements ionisants ayant traversé ledit champ d'observation, ces deux dispositifs de détection étant déplaçables en synchronisme avec les moyens d'émission dans ladite direction de translation,

et que les moyens (17) pour prendre au moins deux images radiographiques à deux dimensions du champ d'observation sont des moyens (17) pour prendre simultanément au moins deux images radiographiques à deux dimensions du champ d'observation, par batayage simultané du champ d'observation avec les moyens d'émission (5, 11 ) et les détecteurs (6, 12) dans la direction de translation (3a).

12. Dispositif selon la revendication 11, dans lequel les moyens d'identification sont adaptés pour identifier des repères de contrôle (C7-C25) visibles et identifiables sur une seule image radiographique, dits repères de contrôle non stéréo-correspondants, et les premiers moyens de reconstruction sont adaptés pour estimer la position géométrique des repères de contrôle non stéréo-correspondants en déplaçant les repères non stéréo-correspondants (C7-C25) du modèle générique, chacun sur une droite joignant :

. d'une part, la source radioactive (5, 6) à l'origine de l'image radiographique où une projection de ce repère de contrôle non stéréo-correspondant est visible et identifiable,

. et d'autre part, ladite projection de ce repère sur l'image radiographique,

les premiers moyens de reconstruction étant adaptés pour déplacer ainsi les repères de contrôle non stéréo-correspondants jusqu'à des positions respectives qui minimisent la déformation globale du modèle générique de l'objet à observer.

13. Dispositif selon la revendication 11 ou la revendication 12, dans lequel les moyens de repérage sont adaptés pour repérer sur les deux images certains des repères de contrôle (C1-C6) qui sont visibles et identifiables sur lesdites deux images, dits "repères de contrôle stéréo-correspondants", et les premiers moyens de reconstruction sont adaptés pour déterminer la position géométrique des repères de contrôle stéréo-correspondants par calcul à partir de mesures de position des projections de ces repères sur les deux images.

14. Dispositif selon l'une quelconque des revendication 11 à 13, dans lequel chaque détecteur (6, 12) comprend une ligne (6a, 12a) de cellules de détection perpendiculaire à la direction de translation (3a), les faisceaux de rayonnements ionisants (10, 16) étant perpendiculaires à ladite direction de translation.

15. Dispositif selon l'une quelconque des revendications 11 à 14, dans lequel les moyens d'émission (5, 11) et les détecteurs (6, 12) sont déplaçables sur une distance de balayage d'au moins 70 cm.

16. Dispositif selon l'une quelconque des revendications 11 à 15, dans lequel les moyens d'émission comprennent deux sources radioactives (5, 11) à l'origine respectivement des deux faisceaux de rayons ionisants (IC, 11).

**Claims**

1. Radiographic imaging method for three-dimensional reconstruction at a low irradiation dose, suitable for calculating a three-dimensional model of at least one pre-determined object (20) to be observed in an observation field (4), this method comprising the following steps:

    (a) taking at least two two-dimensional radiographic images of the observation field, respectively in two non-parallel shooting directions (7, 13),
    (b) locating, on each radiographic image, check marks (C1-C25) belonging to said object to be observed,
    (c) determining a geometric position of each check mark, in a three-dimensional reference frame,
    (d) calculating the three-dimensional shape of a model representing said object from a pre-determined generic model corresponding to said object, this generic model comprising marks that correspond to the check marks identified on the radiographic images, the calculated model being obtained by deformation of the generic model so that said calculated model follows a shape the closest possible to an isometry of the generic model while keeping the marks of the deformed generic model coinciding with the check marks reconstructed at step (c),

**characterised in that**, during step (a), the two radiographic images are taken simultaneously, by scanning, while moving in synchronism in a same translation direction (3a) not parallel to the shooting directions, at least one radioactive source (5, 11) emitting two beams of ionizing rays (10, 16) respectively in the two shooting directions (7, 13).

2. Method according to claim 1, in which:

    - during step (b), some of the identified check marks, referred to as non-stereocorresponding check marks, are visible and identified only on a single image,
    - and during step (c), the geometric position of each non-stereocorresponding check mark (C7-C25) in the three-dimensional reference frame is estimated from the generic model, by moving the non-stereocorresponding check marks of the generic model, each on a straight line joining:

        • on the one hand, the radioactive source (5, 11) at the origin of the radiographic image in which a projection of this non-stereocorresponding check mark is visible and identifiable,
        • and on the other hand, said projection of this mark on the radiographic image,

    the non-stereocorresponding check marks (C7-C25) being thus moved to respective positions that minimize the global deformation of the generic model of the object to be observed.

3. Method according to claim 2, in which, during step (c), the value of the following quadratic sum is minimized:

$$S = \lambda . \sum_{i=1}^{m} k_i . (x_i - x_{i0})^2 ,$$

where $\lambda$ is a constant coefficient, m is an integer number of fictional springs connecting each mark (C1-C25) of the generic model to other marks of this model, $k_i$ is a predetermined stiffness value of the fictional spring of index i, $X_{i0}$ is the length of the fictional spring of index i in the initial generic model, and $x_i$ is the length of the fictional spring of index i in the deformed generic model.

4. Method according to any one of the preceding claims, in which:

- during step (b), at least some of the identifiable check marks are stereocorresponding check marks (C1-C6) visible and identified on the two images,
- and during step (c), the geometric position of the stereocorresponding check marks (C1-C6) is directly calculated from measurements of the position of the projections of these marks on the two images.

5. Method according to claim 1, in which, during step (b), contour lines corresponding to limits of the observed object and/or to lines of greater optical density within said limits are located on each radiographic image, these contour lines comprising projections of the check marks on the radiographic images.

6. Method according to claim 5, in which, during step (c), marks of the generic model corresponding to the check marks are determined, said marks of the generic model comprising portions of said generic model that present tangentially with respect to the rays coming from the radioactive sources and having generated the radiographic images.

7. Method according to claim 6, in which step (c) comprises the following sub-steps:

(c1) creating an adjusted generic model by adapting the size of the generic model and the position of this generic model in the reference frame, so that the respective projections of the adjusted generic model from the two radioactive sources correspond substantially to the two radiographic images,
(c2) selecting marks of the generic model the projections of which on at least one of the radiographic images from the corresponding radioactive source are the closest to the contour lines located during step (b).
(c3) defining an envelope surface formed by rays coming from each radioactive source (5, 11) and having contributed to generating said contour lines of the radiographic images,
(c4) determining some marks of the adjusted generic model corresponding to surfaces of said adjusted generic model that are tangent to said envelope surfaces, the marks of the adjusted generic model thus determined corresponding to the check marks,
(c5) and determining the geometric position of each check mark by projecting the corresponding mark of the adjusted generic model on the corresponding envelope surface.

8. Method according to any one of the preceding claims, in which the two shooting directions (7, 13) are perpendicular to each other.

9. Method according to claim 8, in which each of the radioactive sources (5, 11) is made to emit a beam of ionizing radiations (10, 16) in a plane perpendicular to the translation direction (3a).

10. Method according to any one of the preceding claims, in which the two beams of ionizing rays (10, 16) are emitted respectively by two radioactive sources (5, 11).

11. Radiographic imaging device for implementing a method according to any one of the preceding claims, this device comprising:

- means (17) for taking at least two two-dimensional radiographic images of the observation field,
- identification means (17) for identifying, on each radiographic image, predetermined check marks (C1-C25) belonging to said object to be observed,
- first reconstruction means (17) for determining a geometric position of each check mark in a three-dimensional reference frame (X, Y, Z), from a predetermined generic model corresponding to said object, this generic model comprising marks that correspond to the check marks identified on the radiographic images,
- and second reconstruction means (17) for calculating the three-dimensional shape of a model representing said object from the generic model, said second reconstruction means being suitable for determining the calculated model by deformation of the generic model so that said calculated model follows a shape the closest possible to an isometry of the generic model while keeping the marks (C1-C25) of the deformed generic model coinciding with the check marks reconstructed by the first reconstruction means,

**characterised in that** the device comprises:

- means for emitting ionizing rays comprising at least one radioactive source (5, 11), these emission means being suitable for emitting respectively two beams of ionizing radiations (10, 16) toward an observation field (4) containing at least one object (20) to be observed, in two non-parallel shooting directions (7, 13), said emission means being movable in a translation direction (3a) not parallel to the shooting directions so that the two beams (10, 16) simultaneously scan the observation field,
- at least two detection devices (6, 12) disposed respectively facing the two beams of ionizing rays (10, 16), beyond the observation field (4), in order to measure the ionizing radiations that passed through said observation field, these two detection devices being movable in synchronism with the emission means in said translation direction,

and **in that** the means (17) for taking at least two two-dimensional radiographic images of the observation field are means (17) for simultaneously taking at least two two-dimensional radiographic images of the observation field, by simultaneous scanning of the observation field with the emission means (5, 11) and the detectors (6, 12) in the translation direction (3a).

12. Device according to claim 11, in which the identification means are suitable for identifying check marks (C7-C25) visible and identifiable on a single radiographic image, referred to as non-stereocorresponding check marks, and the first reconstruction means are suitable for estimating the geometric position of the non-stereocorresponding check marks by moving the non-stereocorresponding marks (C7-C25) of the generic model, each on a straight line joining:

- on the one hand, the radioactive source (5, 6) at the origin of the radiographic image where a projection of this non-stereocorresponding check mark is visible and identifiable,
- and on the other hand, said projection of this mark on the radiographic image,

the first reconstruction means being suitable for thus moving the non-stereocorresponding check marks to respective positions that minimize the global deformation of the generic model of the object to be observed.

13. Device according to claim 11 or claim 12, in which the locating means are suitable for locating, on the two images, some of the check marks (C1-C6) that are visible and identifiable on said two images, referred to as "stereocorresponding check marks", and the first reconstruction means are adapted for determining the geometric position of the stereocorresponding check marks by calculation from position measurements of the projections of these marks on the two images.

14. Device according to any one of claims 11 to 13, in which each detector (6, 12) comprises a row (6a, 12a) of detection cells perpendicular to the translation direction (3a), the beams of ionizing radiations (10, 16) being perpendicular to said translation direction.

15. Device according to any one of claims 11 to 14, in which the emission means (5, 11) and the detectors (6, 12) are movable over a scanning distance of at least 70 cm.

16. Device according to any one of claims 11 to 15, in which the emission means comprise two radioactive sources (5, 11) at the origin respectively of the two beams of ionizing rays (10, 11).

**Patentansprüche**

1. Röntgenbildgebungsverfahren zur dreidimensionalen Rekonstruktion mit geringer Bestrahlungsdosis, das dazu geeignet ist, ein dreidimensionales Modell mindestens eines vorbestimmten Objekts (20) zu berechnen, das in einem Betrachtungsfeld (4) betrachtet werden soll, wobei dieses Verfahren die folgenden Schritte umfasst:

(a) Aufnehmen mindestens zwei zweidimensionaler Röntgenbilder des Betrachtungsfelds, jeweils gemäß zwei nicht parallelen Bildaufnahmerichtungen (7, 13),
(b) Markieren von Kontrollmarken (C1-C25) auf jedem Röntgenbild, die zum zu betrachtenden Objekt gehören,
(c) Bestimmen einer geometrischen Position jeder Kontrollmarke in einem dreidimensionalen Bezugssystem,
(d) Berechnen der dreidimensionalen Form eines das Objekt darstellenden Modells ausgehend von einem dem Objekt entsprechenden vorbestimmten generischen Modell, wobei dieses generische Modell Marken aufweist, die den auf den Röntgenbildern identifizierten Kontrollmarken entsprechen, wobei das berechnete Modell durch Verformung des generischen Modells derart erhalten wird, dass das berechnete Modell einer einer Isometrie des generischen Modells nächstmöglichen Form folgt, unter Beibehaltung der Koinzidenz der Marken des verformten generischen Modells mit den im Schritt (c) rekonstruierten Kontrollmarken,

**dadurch gekennzeichnet, dass** während des Schritts (a) die zwei Röntgenbilder gleichzeitig durch Abtasten aufgenommen werden, indem synchron in der gleichen Translationsrichtung (3a) nicht parallel zu den Bildaufnahmerichtungen mindestens eine radioaktive Quelle (5, 11) verschoben wird, die zwei Bündel ionisierender Strahlen (10, 16) in den zwei Bildaufnahmerichtungen (7, 13) emittiert.

2. Verfahren nach Anspruch 1, bei dem:

- während des Schritts (b) bestimmte der identifizierten Kontrollmarken, nicht stereo-korrespondierende Kontrollmarken genannt, nur in einem Bild sichtbar und identifiziert sind,

- und während des Schritts (c) die geometrische Position jeder nicht stereo- korrespondierenden Kontrollmarke (C7- C25) im dreidimensionalen Bezugssystem ausgehend vom generischen Modell geschätzt wird, indem die nicht stereo-korrespondierenden Kontrollmarken des generischen Modells je auf einer Geraden verschoben werden, die:

. einerseits die radioaktive Quelle (5, 11), die Ausgangspunkt des Röntgenbilds ist, auf dem eine Projektion dieser nicht stereo-korrespondierenden Kontrollmarke sichtbar und identifizierbar ist,
. und andererseits die Projektion dieser Marke auf dem Röntgenbild,

verbindet,
wobei die nicht stereo-korrespondierenden Kontrollmarken (C7-C25) so bis in jeweilige Positionen verschoben werden, die die globale Verformung des generischen Modells des zu betrachtenden Objekts minimieren.

3. Verfahren nach Anspruch 2, bei dem während des Schritts (c) der Wert der quadratischen Summe minimiert wird:

$$S = \lambda . \sum_{i=1}^{m} k_i . (x_i - x_{i0})^2$$

wobei $\lambda$ ein konstanter Koeffizient, m eine ganze Anzahl fiktiver Federn, die jede Marke (C1-C25) des generischen Modells mit anderen Marken dieses Modells verbinden, $k_i$ ein vorbestimmter Steifheitswert der fiktiven Feder mit dem Index i, $X_{i0}$ die Länge der fiktiven Feder mit dem Index i im generischen Anfangsmodell und $X_i$ die fiktive Federlänge mit dem Index i im verformten generischen Modell ist.

4. Verfahren nach einem der vorhergehenden Ansprüche, bei dem:

- während des Schritts (b) mindestens bestimmte der identifizierbaren Kontrollmarken stereo-korrespondierende Kontrollmarken (C1-C6) sind, die auf den zwei Bildern sichtbar und identifiziert sind,
- und während des Schritts (c) die geometrische Position der stereo-korrespondierenden Kontrollmarken (C1-C6) direkt ausgehend von Positionsmessungen der Projektionen dieser Marken auf die zwei Bilder berechnet wird.

5. Verfahren nach Anspruch 1, bei dem während des Schritts (b) auf jedem Röntgenbild Umrisslinien markiert werden, die Grenzen des betrachteten Objekts und/oder Linien größerer optischer Dichte innerhalb der Grenzen entsprechen, wobei diese Umrisslinien Projektionen der Kontrollmarken auf die Röntgenbilder enthalten.

6. Verfahren nach Anspruch 5, bei dem während des Schritts (c) Marken des generischen Modells entsprechend den Kontrollmarken bestimmt werden, wobei die Marken des generischen Modells Abschnitte des generischen Modells enthalten, die tangential bezüglich der von den radioaktiven Quellen stammenden und die Röntgenbilder erzeugt habenden Strahlen erscheinen.

7. Verfahren nach Anspruch 6, bei dem der Schritt (c) die folgenden Teilschritte aufweist:

(c1) Erzeugen eines neueingestellten generischen Modells durch Anpassen der Größe des generischen Modells und der Position dieses generischen Modells im Bezugssystem, damit die jeweiligen Projektionen des neueingestellten generischen Modells ausgehend von den zwei radioaktiven Quellen im Wesentlichen den zwei Röntgenbildern entsprechen,
(c2) Auswählen der Marken des generischen Modells, dessen Projektionen auf mindestens eines der Röntgenbilder ausgehend von der entsprechenden radioaktiven Quelle den Umrisslinien am nächsten sind, die während des Schritts (b) markiert wurden,
(c3) Definieren einer Hüllfläche, die von Strahlen geformt wird, welche von jeder radioaktiven Quelle (5, 11) stammen und die dazu beigetragen haben, die Umrisslinien der Röntgenbilder zu erzeugen,
(c4) Bestimmen bestimmter Marken des neueingestellten generischen Modells entsprechend Flächen des neueingestellten generischen Modells, die die Hüllflächen tangieren, wobei die so bestimmten Marken des neueingestellten generischen Modells den Kontrollmarken entsprechen,
(c5) und Bestimmen der geometrischen Position jeder Kontrollmarke durch Projektion der entsprechenden Marke des neueingestellten generischen Modells auf der entsprechenden Hüllfläche.

8. Verfahren nach einem der vorhergehenden Ansprüche, bei dem die zwei Bildaufnahmerichtungen (7, 13) zueinander lotrecht sind.

9. Verfahren nach Anspruch 8, bei dem man jede der radioaktiven Quellen (5, 11) ein Bündel ionisierender Strahlungen (10, 16) in einer Ebene lotrecht zur Translationsrichtung (3a) emittieren lässt.

10. Verfahren nach einem der vorhergehenden Ansprüche, bei dem die zwei Bündel ionisierender Strahlen (10, 16) von zwei radioaktiven Quellen (5, 11) emittiert werden.

11. Vorrichtung zur Röntgenbildgebung zur Anwendung eines Verfahrens nach einem der vorhergehenden Ansprüche, wobei diese Vorrichtung Folgendes aufweist:

    - Einrichtungen (17) zur Aufnahme von mindestens zwei zweidimensionalen Röntgenbildern des Betrachtungsfelds,
    - Identifikationseinrichtungen (17), um auf jedem Röntgenbild vorbestimmte Kontrollmarken (C1-C25) zu identifizieren, die zum zu betrachtenden Objekt gehören,
    - erste Rekonstruktionseinrichtungen (17), um eine geometrische Position jeder Kontrollmarke in einem dreidimensionalen Bezugssystem (X, Y, Z) ausgehend von einem vorbestimmten generischen Modell zu bestimmen, das dem Objekt entspricht, wobei dieses generische Modell Marken aufweist, die den auf den Röntgenbildern identifizierten Kontrollmarken entsprechen,
    - und zweite Rekonstruktionseinrichtungen (17) zum Berechnen der dreidimensionalen Form eines das Objekt darstellenden Modells ausgehend vom generischen Modell, wobei die zweiten Rekonstruktionseinrichtungen dazu geeignet sind, das berechnete Modell durch Verformung des generischen Modells derart zu bestimmen, dass das berechnete Modell einer einer Isometrie des generischen Modells nächstmöglichen Form folgt, unter Beibehaltung der Koinzidenz der Marken (C1-C25) des verformten generischen Modells mit den von den ersten Rekonstruktionseinrichtungen rekonstruierten Kontrollmarken,

    **dadurch gekennzeichnet, dass** die Vorrichtung Folgendes aufweist:

    - Einrichtungen zum Emittieren von ionisierenden Strahlen, die mindestens eine radioaktive Quelle (5, 11) enthalten, wobei diese Emissionseinrichtungen dazu geeignet sind, je zwei Bündel ionisierender Strahlungen (10, 16) in zwei nicht parallelen Bildaufnahmerichtungen (7, 13) zu einem Betrachtungsfeld (4) zu emittieren, das mindestens ein zu betrachtendes Objekt (20) enthält, wobei die Emissionseinrichtungen gemäß einer Translationsrichtung (3a) nicht parallel zu den Bildaufnahmerichtungen verschiebbar sind, damit die zwei Bündel (10, 16) das Betrachtungsfeld gleichzeitig abtasten,
    - mindestens zwei Erfassungsvorrichtungen (6, 12), die je gegenüber den zwei Bündeln ionisierender Strahlen (10, 16) jenseits des Betrachtungsfelds (4) angeordnet sind, um die ionisierenden Strahlungen zu messen, die das Betrachtungsfeld durchquert haben, wobei diese zwei Erfassungsvorrichtungen synchron mit den Emissionseinrichtungen in der Translationsrichtung verschiebbar sind,

    und dass die Einrichtungen (17) zur Aufnahme von mindestens zwei zweidimensionalen Röntgenbildern des Betrachtungsfelds Einrichtungen (17) zur gleichzeitigen Aufnahme von mindestens zwei zweidimensionalen Röntgenbildern des Betrachtungsfelds sind, und eine gleichzeitige Abtastung des Betrachtungsfelds mit den Emissionseinrichtungen (5, 11) und den Detektoren (6, 12) in der Translationsrichtung (3a) aufweisen.

12. Vorrichtung nach Anspruch 11, bei der die Identifikationseinrichtungen dazu geeignet sind, auf einem einzigen Röntgenbild sichtbare und identifizierbare Kontrollmarken (C7-C25) zu identifizieren, nicht stereo-korrespondierende Kontrollmarken genannt, und die ersten Rekonstruktionseinrichtungen dazu geeignet sind, die geometrische Position der nicht stereo-korrespondierenden Kontrollmarken zu schätzen, indem die nicht stereo-korrespondierenden Marken (C7-C25) des generischen Modells je auf einer Gerade verschoben werden, die:

    . einerseits die radioaktive Quelle (5, 6) am Ursprung des Röntgenbilds, wo eine Projektion dieser nicht stereo-korrespondierenden Kontrollmarke sichtbar und identifizierbar ist,
    . und andererseits die Projektion dieser Marke auf das Röntgenbild,

    verbindet,
    wobei die ersten Rekonstruktionseinrichtungen dazu geeignet sind, so die nicht stereo-korrespondierenden Kontrollmarken bis in jeweilige Positionen zu verschieben, die die globale Verformung des generischen Modells des zu betrachtenden Objekts minimieren.

13. Vorrichtung nach Anspruch 11 oder Anspruch 12, bei der die Markierungseinrichtungen dazu geeignet sind, auf den zwei Bildern bestimmte Kontrollmarken (C1-C6) zu markieren, die auf den zwei Bildern sichtbar und identifizierbar sind, "stereo-korrespondierende Kontrollmarken" genannt, und die ersten Rekonstruktionseinrichtungen dazu geeignet sind, die geometrische Position der stereo-korrespondierenden Kontrollmarken durch Berechnung ausgehend von Positionsmessungen der Projektionen dieser Marken auf die zwei Bilder zu bestimmen.

14. Vorrichtung nach einem der Ansprüche 11 bis 13, bei der jeder Detektor (6, 12) eine Reihe (6a, 12a) von Erfassungszellen lotrecht zur Translationsrichtung (3a) enthält, wobei die Bündel von ionisierenden Strahlungen (10, 16) lotrecht zur Translationsrichtung sind.

15. Vorrichtung nach einem der Ansprüche 11 bis 14, bei der die Emissionseinrichtungen (5, 11) und die Detektoren (6, 12) über eine Abtaststrecke von mindestens 70 cm verschiebbar sind.

16. Vorrichtung nach einem der Ansprüche 11 bis 15, bei der die Emissionseinrichtungen zwei radioaktive Quellen (5, 11) enthalten, die je am Ursprung der zwei Bündel ionisierender Strahlen (10, 11) sind.

FIG.1.

FIG.2.

FIG.5.

FIG.3.

FIG.4.

## RÉFÉRENCES CITÉES DANS LA DESCRIPTION

**Documents brevets cités dans la description**

- FR 2749402 A

- FR 2754068 A

**Littérature non-brevet citée dans la description**

- **Aziz et al.** Direct linear transformation from comparator coordinates into object space coordinates in close range photogrammetry. *Prcc. ASP/UI Symp. Close Range Photogrammetry,* 1971
- **Marzan.** Rational design for close range photogrammetry. *PhD thesis, Department of Civil Engineering,* 1976

- **André et al.** Optimized vertical stereo base radiographic setup for the clinical three-dimensional reconstruction of the human spine. *J. Biomech.,* 1994, vol. 27, 1023-1035
- **Trochu.** A contouring program based on dual kriging interpolation. *Eng. Comput.,* 1993, vol. 9, 160-177
- **MITTON et al.** *Medical & Biological Engineering & Computing,* 2000, vol. 38, 133-139